# EUROPEAN PATENT SPECIFICATION

(11) **EP 4 615 316 B1**
(45) Date of publication and mention of the grant of the patent: **14.10.2026**
(21) Application number: 23813476.1
(22) Date of filing: 07.11.2023
(51) Int. Cl.: A61B 5/06, A61B 5/00, A61B 1/00, A61B 34/30

(54) **SYSTEM AND METHOD FOR CALIBRATING SENSORS OF AN ENDOSCOPE AND RELATED CALIBRATION DEVICE**
SYSTEM UND VERFAHREN ZUR KALIBRIERUNG VON SENSOREN EINES ENDOSKOPS UND ZUGEHÖRIGE KALIBRIERUNGSVORRICHTUNG
SYSTÈME ET PROCÉDÉ D'ÉTALONNAGE DE CAPTEURS D'ENDOSCOPE ET DISPOSITIF D'ÉTALONNAGE ASSOCIÉ

(30) Priority: 10.11.2022 IT 202200023199
(43) Date of publication of application: 17.09.2025
(73) Proprietor: Atlas Endoscopy Limited, Leeds LS2 3AA (GB)
(72) Inventor: SCAGLIONI, Bruno, Leeds Yorkshire LS16DP (GB); MAZZEO, Donato, Leeds Yorkshire LS16DP (GB); VALDASTRI, Pietro, Leeds Yorkshire LS16DP (GB)
(74) Representative: Pennacchio, Salvatore Giovanni
(86) International application number: PCT/IB2023/061213
(87) International publication number: WO 2024/100539

(56) References cited:
- WO-A1-2022/193411
- JP-A- 2014 100 233
- US-A1- 2021 251 473
- US-B2- 11 122 965
- MOHAMMED IBRAHIM K ET AL: "Design and implementation of positioning and control systems for capsule endoscopes", IET SCIENCE, MEASUREMENT AND TECHNOLOGY, THE INSTITUTION OF ENGINEERING AND TECHNOLOGY, MICHAEL FARADAY HOUSE, SIX HILLS WAY, STEVENAGE, HERTS. SG1 2AY, UK, vol. 14, no. 7, 1 September 2020 (2020-09-01), pages 745 - 754, XP006092103, ISSN: 1751-8822, DOI: 10.1049/IET-SMT.2019.0223
- TRUNG DUC THAN ET AL: "A Review of Localization Systems for Robotic Endoscopic Capsules", IEEE TRANSACTIONS ON BIOMEDICAL ENGINEERING, IEEE, USA, vol. 59, no. 9, 1 September 2012 (2012-09-01), pages 2387 - 2399, XP011456988, ISSN: 0018-9294, DOI: 10.1109/TBME.2012.2201715

## Description

### Field of application

The present invention generally relates to magnetically guided endoscopic systems usable in the medical field. In particular, the invention relates to a system and method for calibrating sensors accommodated in an endoscope usable in a magnetically guided endoscopic system.

The present invention also relates to a calibration device usable to perform the aforesaid method.

### Prior art

As known, a magnetically guided endoscopic robotic system consists of a robotic platform supporting in the end part thereof one or more electronically controllable magnetic field sources, in terms of generated field and/or position and orientation of the field itself, and of a capsular endoscopic element, for example connected by means of an electrical connection (wired), for example an electrical wire or cable, to the robotic platform, containing a magnetic field source therein.

Such a capsular endoscopic element or endoscope is insertable into a patient's natural cavity, for example in the gastrointestinal tract, through the natural sphincters to perform a diagnostic assessment on the patient. In particular, under the action of the magnetic field generated by the one or more controllable magnetic field sources of the robotic platform, it is possible to orient, locate and control the movement of the endoscope within the patient's gastrointestinal tract.

US 11,122,965 B2 and other similar known technical solutions describe an endoscopic capsule of known type usable in a magnetically guided endoscopic system. Such an endoscopic capsule comprises a permanent magnet inside the capsule and a plurality of sensors, including:
- one or more magnetic field sensors (MFS), for example one or more Hall-effect sensors;
- an Inertial Measurement Unit (IMU) which generally comprises an accelerometer and/or a gyroscope.

An endoscopic capsule can further comprise a camera, usable to allow an operator to view sections of the patient's gastrointestinal tract and to manually control the movement of the capsule, in addition to enabling any automatic image processing, such as automatic learning and navigation algorithms.

Each sensor has its a reference system thereof which indicates the direction of the measured values with respect to the sensor body.

However, regardless of the manufacturing quality, each of the sensors equipping the endoscope suffers from positioning and sensitivity inaccuracies which can degrade the quality of the measurement made.

For example, the mounting/orientation position of the accelerometer is not perfectly predictable, thus the axis of such a sensor could be inclined with respect to the overall orientation of the endoscope.

The gyroscope has the same drawback. Moreover, as known, gyroscopes are affected by a distortion which changes over time due to production features and temperature.

In addition, Hall-effect magnetic field sensors are rigidly connected to the magnet inside the endoscope, thus it is not possible to prevent them from also detecting the magnetic field generated by the latter. Such a detected magnetic field forms a constant offset value, known only after assembly, which must be measured and removed from the readings returned by such sensors.

For the same reason, the measurements returned by the magnetic field sensors can be affected by distortions given by the earth's magnetic field which must be eliminated.

Based on the above drawbacks, it is clear that the sensors equipping the endoscope of a magnetically guided endoscopic system require calibration before using the endoscope itself.

Nowadays, several calibration solutions have been suggested for endoscopic devices for medical use which use magnetic interaction. However, there are no solutions specifically designed for the simultaneous and integrated calibration of inertial sensors and magnetic sensors in the presence of a permanent magnet on board the endoscope.

Therefore, the need to devise a calibration solution for an endoscope of a magnetically guided endoscopic system which allows overcoming the limitations and drawbacks of the traditional methodologies remains strongly felt.

### SUMMARY OF THE INVENTION

Therefore, it is the object of the present invention to provide a system and method for calibrating the sensors accommodated in an endoscope usable in a magnetically guided endoscopic system, which allows at least partially overcoming the limitations of the known solutions and which, by using magnetic interaction, allows simultaneously manipulating and locating the endoscope during a diagnostic assessment on the patient in a reliable and simple manner.

Such an object is achieved by a calibration system of the sensors accommodated in an endoscope according to claim 1.

The present invention also relates to a method of calibrating the sensors accommodated in an endoscope implemented by the aforesaid system according to claim 17.

Preferred and advantageous embodiments of the method of calibrating the sensors accommodated in an endoscope and of the related calibration system are the subject of the dependent claims.

### BRIEF DESCRIPTION OF THE DRAWINGS

Further features and advantages of the invention will be apparent from the following description of preferred embodiments thereof, given by way of nonlimiting indication, with reference to the accompanying drawings, in which:
- **figure 1** schematically shows a first embodiment of a calibration system of sensors accommodated in an endoscope of the present invention;
- **figures 2A, 2B, 2C** show a front view, a front perspective view and a rear perspective view, respectively, of a calibration device usable in the system in figure 1;
- **figure 3** schematically shows a further embodiment of a controllable magnetic field source usable in the system in figure 1;
- **figure 4** schematically shows an example of an actuation mechanism associable with the calibration device of the system in figure 1;
- **figure 5** schematically shows a first embodiment of a calibration value storage unit of the system in figure 1;
- **figure 6** schematically shows a second embodiment of a calibration value storage unit of the system in figure 1;
- **figure 7** schematically shows a second embodiment of a calibration system of the sensors accommodated in an endoscope of the present invention, with the endoscope positioned in a first calibration operating position with respect to a robotic platform of the system;
- **figure 8** schematically shows the calibration system in figure 7, with the endoscope positioned in a second operating calibration position with respect to the robotic platform of the system;
- **figures 9A, 9B, 9C****,** **9D, 9E** show a front perspective view, a front view, a rear view, a top view and a bottom view, respectively, of a calibration device usable in the system in figure 7;
- **figure 10** shows a perspective view and in open configuration of the calibration device in figure 9A;
- **figure 11** schematically shows a perspective view of a robotic platform usable in the system in figure 7;
- **figure 11a** shows an enlargement of a portion of the robotic platform in figure 11 and of an interface element housable in the robotic platform to allow coupling the calibration device in figure 9A to the robotic platform;
- **figures 12A, 12B** show a front perspective view and a top view, respectively, of the interface element in figure 11a;
- **figures 13A, 13B, 13C** show front perspective views of an assembly of the interface element housable in the robotic platform and of the calibration device in figure 9A, in which such a calibration device is coupled to the interface element along three different mutually orthogonal orientation directions;
- **figure 14** shows, with a flow diagram, the operating steps of the method of calibrating the sensors accommodated in an endoscope of the present invention;

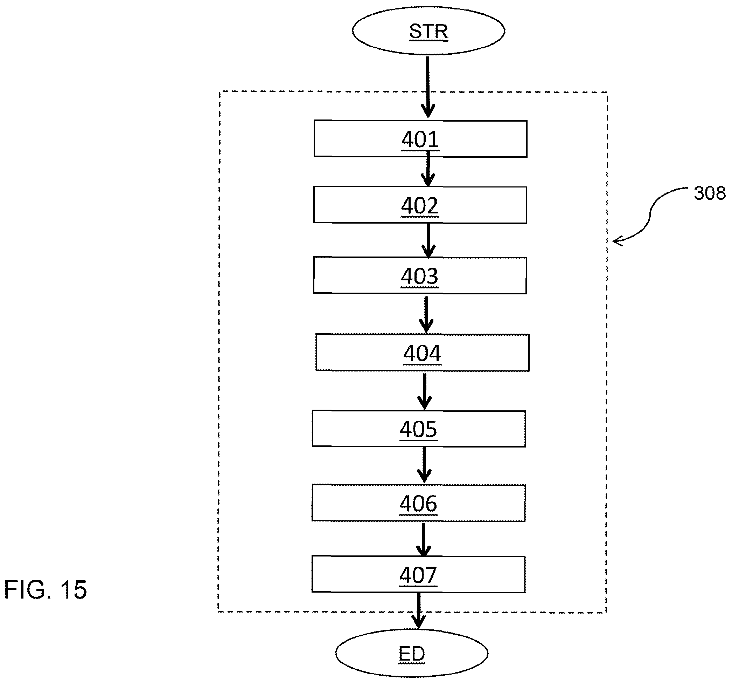

- **figure 15** shows, with a flow diagram, the operating steps in detail of a calibration step of the method in figure 14.

Similar or equivalent elements in the aforesaid figures are indicated by the same reference numerals.

### DETAILED DESCRIPTION

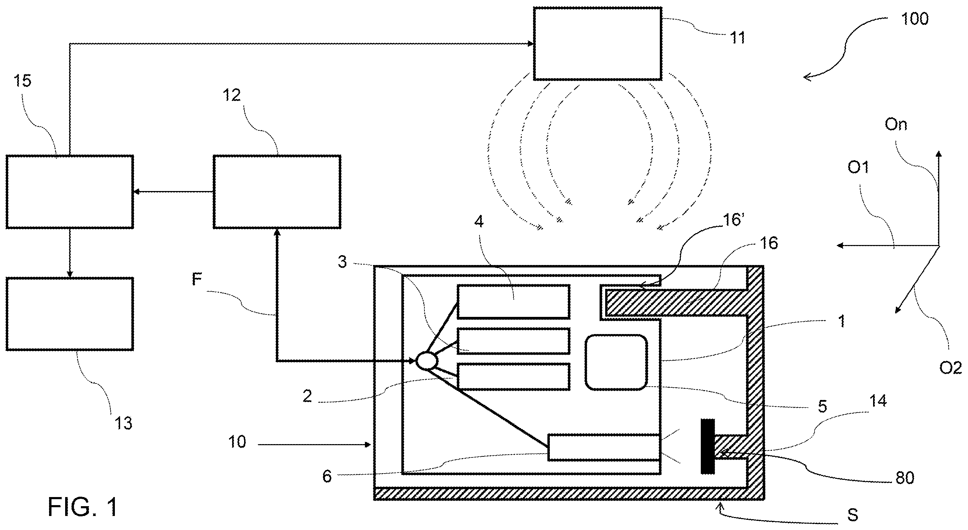

With reference to figures 1, 3-8, an example of a system in accordance with the invention for calibrating sensors accommodated in an endoscope 1 usable in a magnetically guided endoscopic system is indicated as a whole with reference numerals 100, 200 in two embodiments.

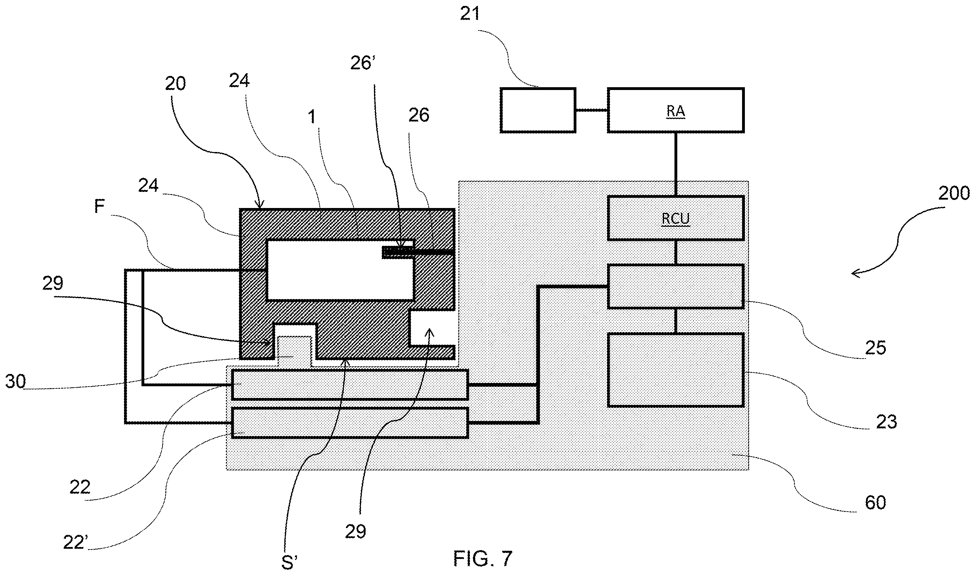

The aforesaid magnetically guided endoscopic system consists, as known, of a robotic platform, for example the robotic platform 60 diagrammatically shown in figures 7-8 and figure 11, which supports in the end part 21 thereof one or more electronically controllable magnetic field sources and a capsular endoscopic element or endoscope 1. Such an endoscope is, for example, connected by means of an electric cable F to the robotic platform 60 and comprises a permanent magnetic field source therein, in particular a permanent magnet 5. The aforesaid endoscope 1 is insertable into a natural cavity of a patient, for example into the patient's gastrointestinal tract, to perform a diagnostic assessment on the patient.

The system 100, 200 for calibrating sensors of an endoscope 1 of the invention is hereinafter also referred to as a calibration system or, more simply, a system.

Such a calibration system 100, 200 comprises the above-mentioned endoscope 1 including one or more sensors 2, 3, 4, 6 to be calibrated. Such an endoscope 1 further includes a permanent magnet 5.

In an exemplary embodiment, such one or more sensors of the endoscope 1 comprise:
- an accelerometer 2,
- a gyroscope 3,
- one or more magnetic field sensors 4 (MFS) operatively associated with the permanent magnet 5 housed inside the endoscope 1. Such magnetic field sensors are, for example, one or more Hall-effect sensors.

In a further embodiment, the aforesaid one or more sensors of the endoscope 1 further comprise a camera 6.

The calibration system 100, 200 further comprises an electronic processing unit 15, 25, for example a microprocessor (Central Processing Unit or CPU), and a magnetic field source 11, 21 controlled by the electronic processing unit 15, 25 to provide a reference magnetic field value to the endoscope 1.

The calibration system 100, 200 further comprises a reading unit 12, 22 connected to the endoscope 1 and configured to acquire data representative of measurements made by the aforesaid one or more sensors 2, 3, 4, 6 of the endoscope to be sent to the electronic processing unit 15, 25.

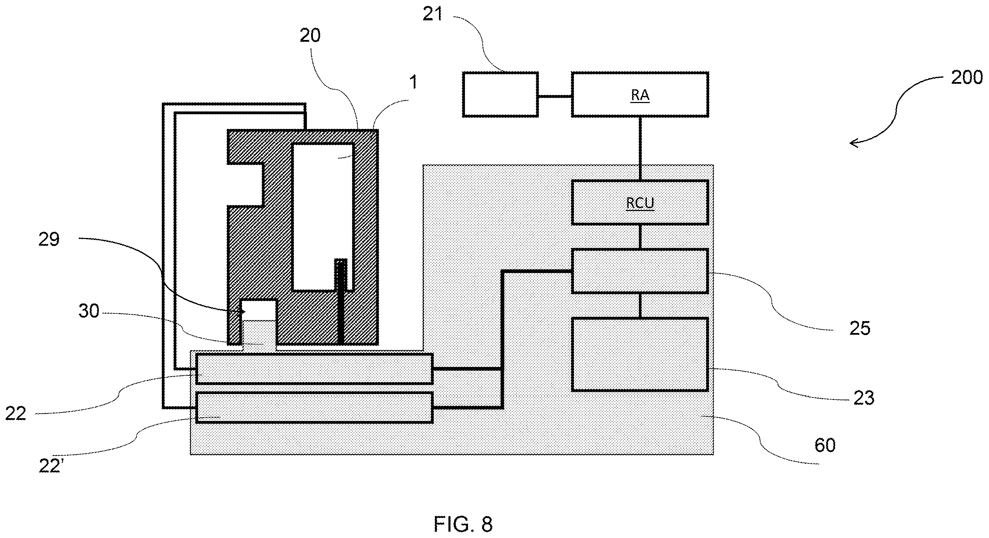

In the exemplary embodiment in figures 1 and 8, such a reading unit 12, 22 is an electronic unit outside the endoscope 1 and operates to acquire the measurement data from the sensors 2, 3, 4, 6 in analog form and to convert them into corresponding digital data to be transferred to the electronic processing unit 15, 25 configured to process them.

In a different embodiment (not shown in the figures), such a reading unit 12, 22 is a unit equipping the endoscope 1.

The system 100 200 of the invention further advantageously comprises a calibration device 10, 20 having a body 14, 24 adapted to house the endoscope 1 and reversible coupling means 16, 16', 26, 26' for reversibly coupling the endoscope 1 to the body 14, 24 of the calibration device 10, 20.

In particular, such reversible coupling means 16, 16', 26, 26' are configured to couple and make the endoscope 1 integral with the body of the calibration device 10, 20 preventing a mutual movement thereof.

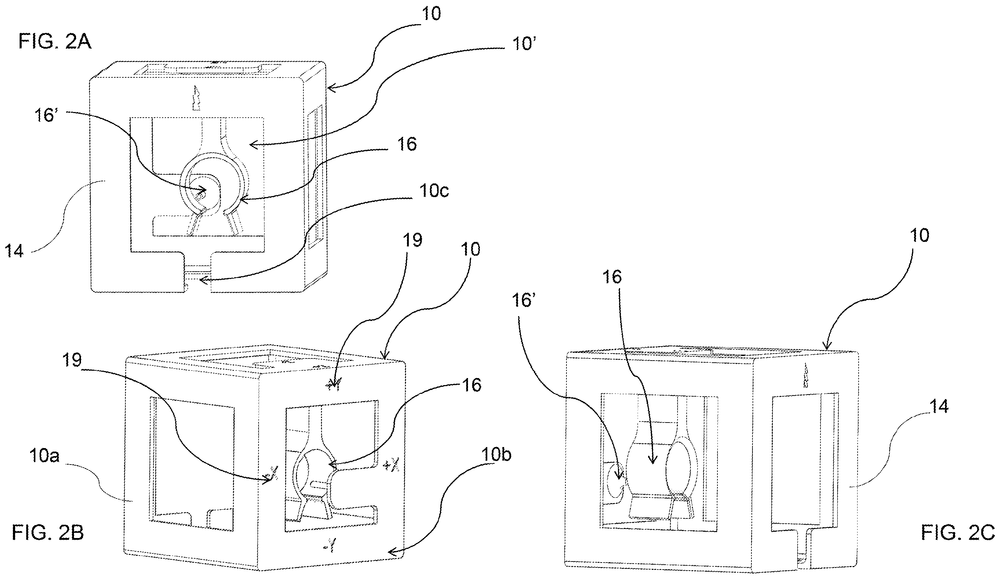

In more detail, with reference to the example of calibration device 10 in figures 2A, 2B, 2C, such reversible coupling means 16, 16' for reversibly coupling the endoscope 1 to the body 14 of the calibration device 10 comprise a mechanism 16 for reversibly locking the endoscope 1 to such a body 14 and a mechanism 16' adapted to fix the orientation of the endoscope with respect to the body 14 of the calibration device 10.

Such a reversible locking mechanism 16, for example jaw-shaped as in the figures, is configured to lock and hold the endoscope 1 in position in the calibration device 10, in a closed jaw configuration, and to release the endoscope 1 in an open jaw position.

Note that the body 14, 24 of the calibration device 10, 20 comprises an external reference surface S, S' oriented, with respect to the magnetic field source 11, 21, in a first direction O1 of a plurality of predetermined directions O1, 02,..., On.

In other words, the reference surface S, S' is oriented in the first direction O1 when said direction O1 is parallel to the aforesaid surface.

For example, such predetermined directions O1, 02,..., On can comprise both the three directions defined by the axes X, Y, Z of an orthogonal Cartesian reference system and also other directions defined by further reference axes X', Y', Z' obtained by rotating the axes X, Y, Z of the orthogonal Cartesian reference system by one or more predetermined angles.

Moreover, the body 14, 24 of the calibration device 10, 20 is movable to orient in sequence, with respect to the magnetic field source 11, 21, the external reference surface S, S' from the first direction O1 in one or more second directions 02, ..., On of the plurality of predetermined directions O1, 02, ..., On.

In particular, the electronic processing unit 15, 25 is configured to acquire the data representative of the measurements made by said one or more sensors 2, 3, 4, 6 of the endoscope 1 when the external reference surface S, S' of the body 14, 24 of the calibration device 10, 20 is oriented both in the first direction O1 and in each of the second directions of the plurality of predetermined directions O1, 02,..., On.

Moreover, the electronic processing unit 15, 25 is configured to perform a calibration of said one or more sensors 2, 3, 4, 6 of the endoscope 1 based on the acquired data.

In an exemplary embodiment, the calibration device 10, 20 of the system 100, 200 comprises means 19, 29 for identifying that said external reference surface S, S' of the body 14, 24 is oriented in the first direction O1 and in each of the aforesaid second directions of the plurality of predetermined directions O1, 02,..., On.

With reference to the example of calibration device 10 in figures 2A, 2B, 2C, such a calibration device 10 has the shape of a prism, for example with a square or rectangular base, having an internal cavity 10' for housing the endoscope 1 delimited by a frame 10a defining a plurality of mutually orthogonal flat surfaces 10b of the calibration device 10.

The jaw-shaped reversible locking mechanism 16 and the mechanism for fixing the orientation 16' of the endoscope 1 are, for example, fixed to such a frame 10a. In particular, the reversible locking mechanism 16 protrudes inside the aforesaid internal cavity 10'.

In such an example, the means 19 for identifying that said external reference surface S of the body 14 is oriented in the first direction O1 and in each of the aforesaid second directions of the plurality of predetermined directions O1, O2,..., On comprise marks imprinted on at least two of such flat surfaces 10b of the frame 10a.

The frame 10a in figures 2A-2C comprises a slot or interruption portion 10c to allow the passage of the electric cable F connecting the endoscope 1 to the robotic platform.

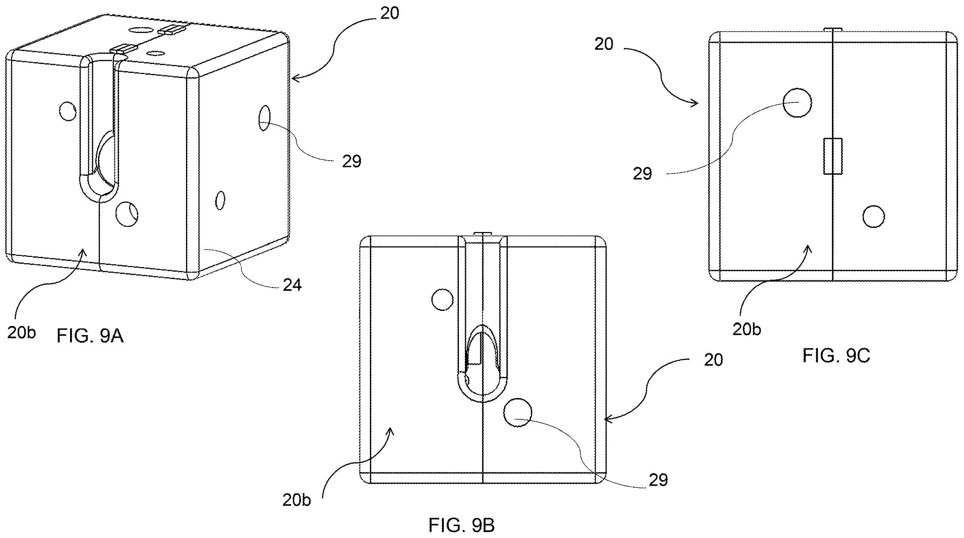

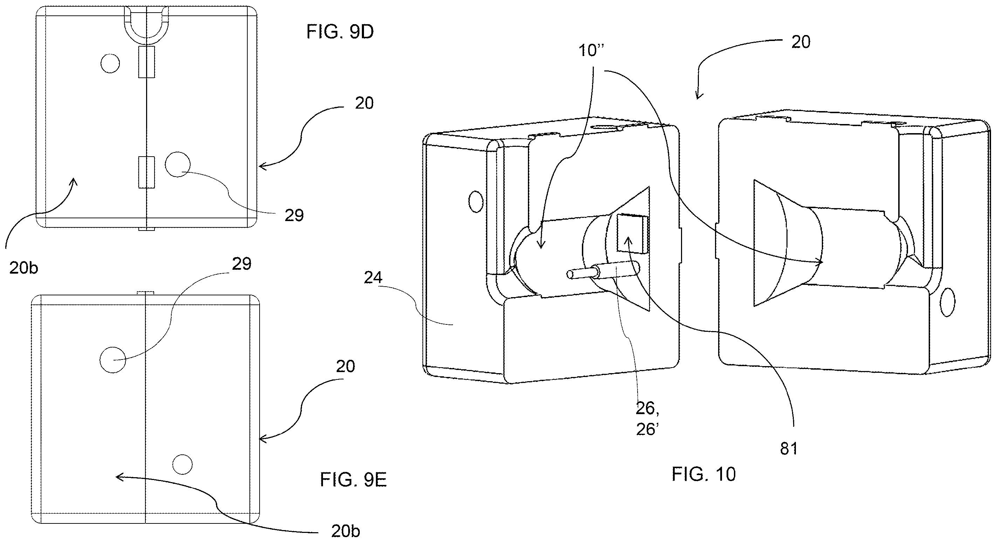

With reference to the embodiment in figures 9A-9E and 10, the calibration device 20 of the invention has the body 24 having a prism shape, for example with a square or rectangular base, adapted to delimit an internal cavity 10" of the body for housing the endoscope 1. Such a body 24 defines six mutually orthogonal flat surfaces 20b of the calibration device 20.

The reversible locking mechanism 26 also operates as an element for fixing the orientation 26' of the endoscope 1 and is configured as a protruding pin, in an off-center position with respect to a median axis of the body 24 of the calibration device 20, in the aforesaid internal cavity 10" for housing the endoscope 1.

In this case, the means 29 for identifying that the external reference surface S' of the body 24 is oriented in the first direction O1 and in each of the aforesaid second directions of the plurality of predetermined directions O1, 02,..., On comprise one or more holes 29, in particular cylindrical holes of a non-through type and with differentiated diameters, obtained on one or more of such flat surfaces 20b of the body 24 of the calibration device 20.

For example, each of the flat surfaces 20b of the calibration device 20 which can act as an external reference surface S' comprises two cylindrical holes 29 having a different diameter from each other and obtained in different positions of such a flat surface 20b.

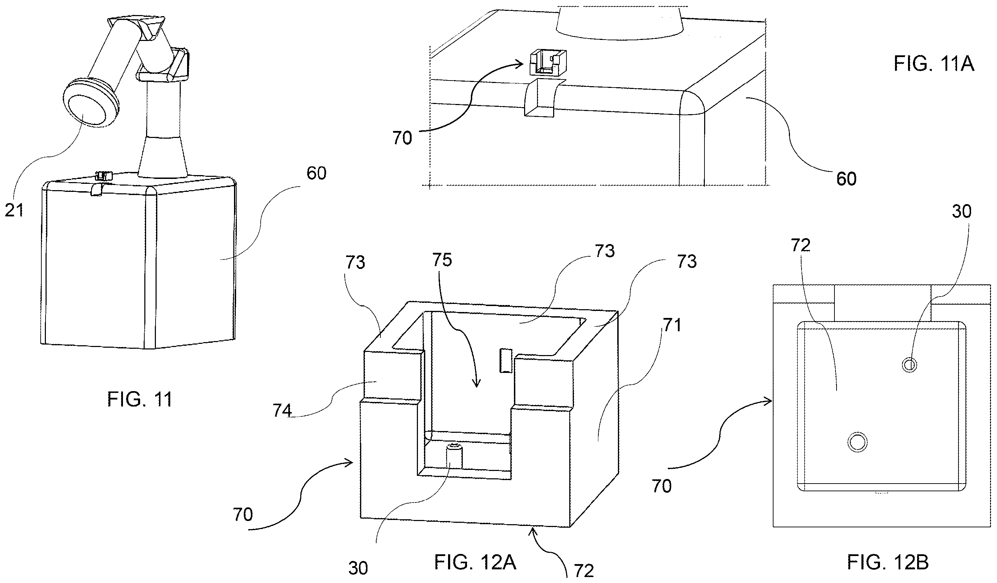

Such one or more holes 29 are configured to engage respective one or more protruding pins 30 associated with the robotic platform 60 of the calibration system 200 shown in figure 11, as will be clarified below.

In an embodiment, with reference to figures 1, 7-8, the calibration system 100, 200 of the invention further comprises a storage unit 13, 23, controlled by the electronic processing unit 15, 25, for example a solid-state drive (SSD), for storing calibration values associated with the aforesaid one or more sensors 2, 3, 4, 6.

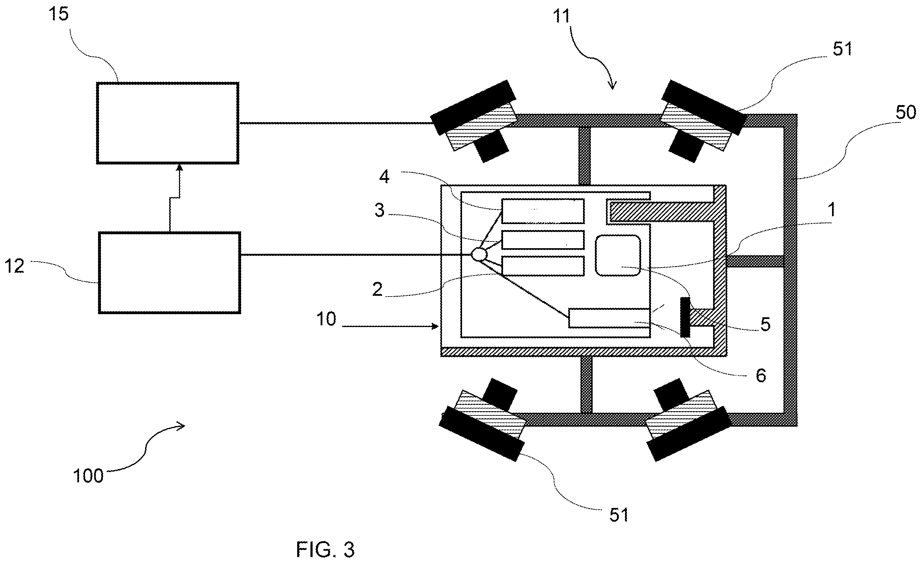

In an embodiment of the calibration system 100, with reference to figure 3, the calibration device 10 is housed inside a calibration frame 50 and the magnetic field source 11 controlled by the electronic processing unit 15 comprises a plurality of coils 51 fixed to the frame 50 adapted to provide one or more reference magnetic field values to the endoscope 1.

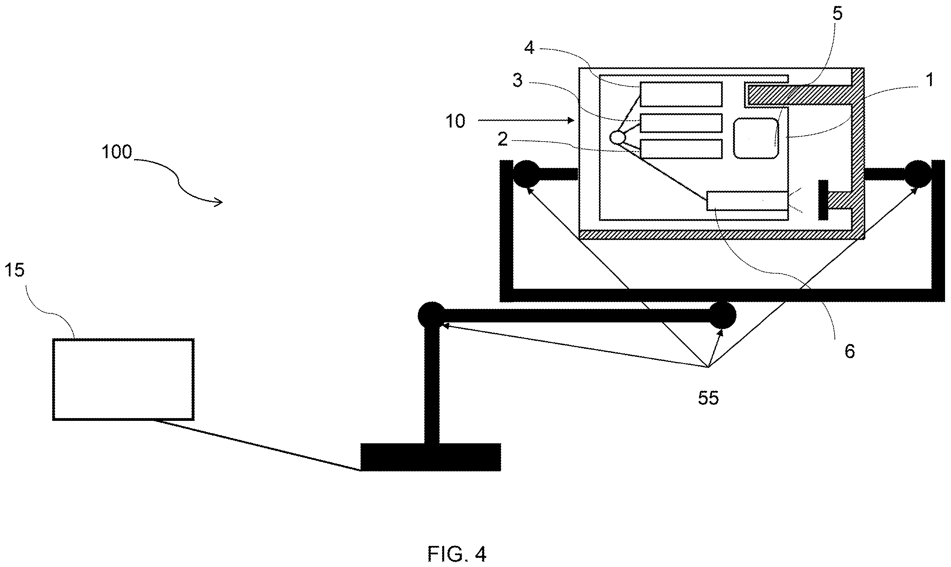

In a further embodiment of the calibration system 100, with reference to figure 4, the calibration device 10 is connected to a rotation actuation mechanism 55 articulated on three axes, controlled by the electronic processing unit 15 to allow the automatic rotation of the endoscope 1 about any of such axes so as to take three or more mutually orthogonal positions.

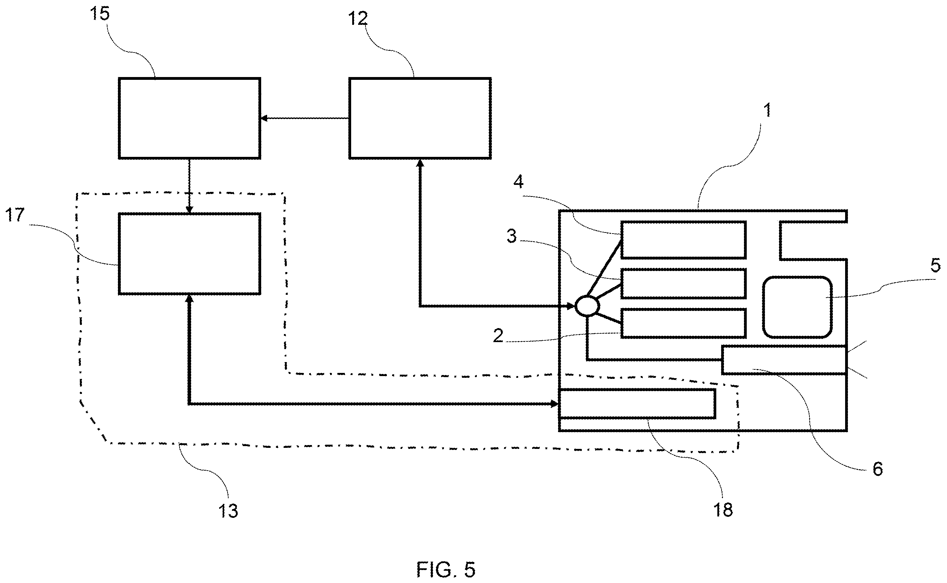

In a further embodiment of the calibration system 100, with reference to figure 5, the endoscope 1 further comprises a non-volatile type memory 18, for example a respective solid-state drive (SSD). Moreover, the unit 13 for storing calibration values associated with the one or more sensors 2, 3, 4, 6 of the endoscope 1 comprises a unit 17 for writing such calibration values in such a non-volatile memory 18.

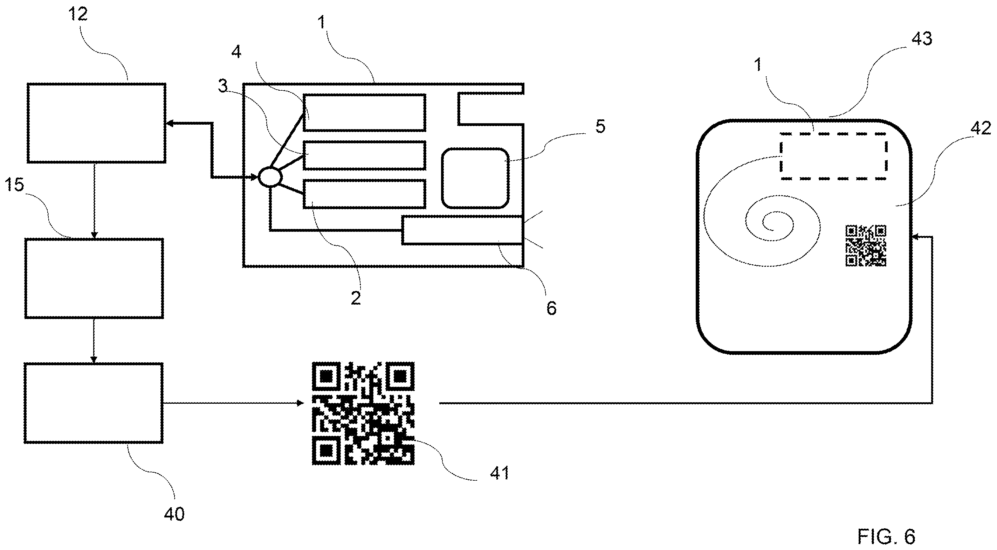

In a further embodiment of the calibration system 100, with reference to figure 6, the aforesaid unit 13 for storing calibration values associated with said one or more sensors 2, 3, 4, 6 of the endoscope 1 further comprises a unit 40 for generating unique graphic identification codes 41. Each of such codes includes the calibration values in a machine-readable format.

In particular, such unique graphic identification codes 41 can be one-dimensional codes such as barcodes, or two-dimensional codes of the Data Matrix or QR-code type.

Moreover, such identification codes 41 are printable on an external surface 42 of a packaging 43 of the endoscope 1.

Note that the calibration system 100 and the related calibration device 10 described above are configured to implement a method of calibrating the sensors housed in the endoscope 1 performed after the manufacturing steps of the endoscope 1 itself.

As mentioned above, with reference to figures 7-8, the calibration system 200 comprises the robotic platform 60 forming the magnetically guided endoscopic system.

In addition to the structural components mentioned above, such a robotic platform 60 can also comprise a calibration value reading unit 22' connected to the endoscope 1 and controlled by the electronic processing unit 25.

Such an electronic processing unit 25 is configured to control a respective control unit RCU of a robotic arm. Such a robotic arm control unit RCU is configured to move a robotic arm RA supporting the controlled magnetic field source 21 outside the endoscope 1.

With reference to figures 11, 11A, 12A-12B, the calibration system 200 of the invention further comprises an interface element 70 housed in the aforementioned robotic platform 60. Such an interface element 70 comprises the aforesaid one or more protruding pins 30 adapted to be engaged with the one or more holes 29 of the calibration device 20 to allow coupling the calibration device 20 to the interface element.

In greater detail, such an interface element 70 has a respective prism-shaped body 71, for example with a square or rectangular base, comprising a base wall 72 connected to side walls 73, 74 orthogonal to the base portion 72 adapted to delimit a first cavity 75 for inserting the calibration device 20 into the interface element 70.

Such one or more protruding pins 30 of the interface element 70 are arranged on the base wall 72 to project towards the first cavity 75.

For example, such protruding pins 30 are cylindrical having a different diameter from each other and obtained in different off-center positions in such a base wall 72.

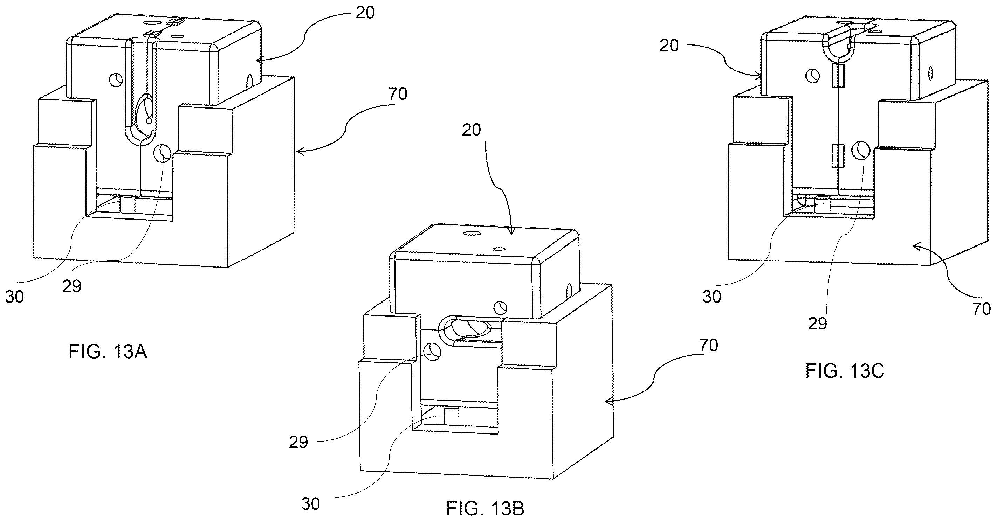

Figures 13A, 13B, 13c show front perspective views of an assembly of the interface element 70 housable in the robotic platform 60 and the calibration device 20, where such a calibration device is coupled to the interface element along three different mutually orthogonal orientation directions. In this case, the location and size of the pins 30 in the interface device 70 and of the respective holes 29 in the calibration device 20 allows the calibration device 20 to be inserted into the interface element only along three mutually orthogonal different orientation directions, each with two possible sides.

Note that the calibration system 200 and the related calibration device 20 described above are configured to implement a method of calibrating the sensors housed in the endoscope 1 which can be performed before the use of the endoscope itself.

With reference to the examples in figures 1 and 10, the calibration device 10, 20 of the invention internally comprises the body 14, 24 housing the endoscope 1, an element 80, 81 for displaying one or more calibration images to be made available to the camera 6 of the endoscope 1.

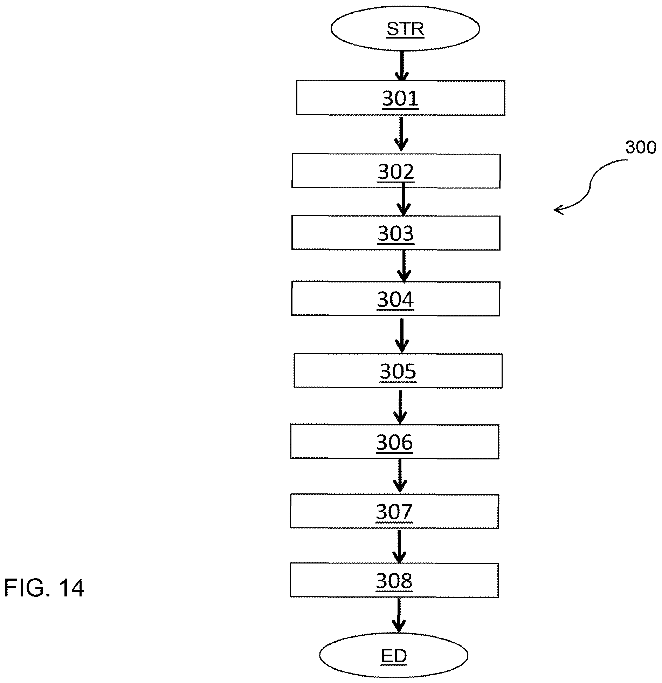

With reference to figure 14, a general embodiment of a method 300 for calibrating sensors accommodated in an endoscope 1 usable in a magnetically guided endoscopic system of the invention is illustrated with a flow diagram.

The method starts with a symbolic start step STR and ends with a symbolic end step ED and in the most general form can be implemented with both the system 100 at the factory and the system 200 before use of the endoscope.

In an embodiment, the electronic processing unit 15, 25 of the system 100, 200 is arranged to execute the codes for an application program implementing the method 300 of the present invention.

In particular, the microprocessor of such a processing unit is configured to load, in a respective memory block, and execute the codes of the application program implementing the method 300 of the present invention.

The calibration method or simply method 300 comprises an initial step of providing 301 a calibration system 100, 200 similar to that described above, i.e., comprising:
- an endoscope 1 including one or more sensors 2, 3, 4, 6 to be calibrated;
- an electronic processing unit 15, 25;
- a magnetic field source 11, 21 controlled by the electronic processing unit 15, 25 to provide a reference magnetic field value to the endoscope 1;
- a reading unit 12, 22 connected to the endoscope 1 and configured to acquire data representative of measurements made by said one or more sensors of the endoscope to be sent to the electronic processing unit 15, 25.

The calibration method 300 further comprises the steps of:
- providing 302 a calibration device 10, 20 having a body 14, 24 adapted to house the endoscope 1;
- coupling and making integral 303 the endoscope 1 with the body 14, 24 of the calibration device 10, 20 preventing a mutual movement thereof by reversible coupling means 16, 16', 26, 26' for reversibly coupling the endoscope 1 to the body of the calibration device 10, 20.

The method 300 also comprises a step of orienting 304, with respect to the aforesaid magnetic field source 11, 21, an external reference surface S, S' of the body 14, 24 of the calibration device 10, 20 in a first direction O1 of a plurality of predetermined directions O1, 02, ..., On.

The method 300 also includes a step of acquiring 305, by the electronic processing unit 15, 25, the data representative of the measurements made by such one or more sensors 2, 3, 4, 6 of the endoscope 1 when the external reference surface S, S' of the body 14, 24 of the calibration device 10, 20 is oriented in the first direction O1.

Moreover, the method 300 includes a step of moving 306 the body 14, 24 of the calibration device 10, 20 to orient in sequence, with respect to the magnetic field source 11, 21, the external reference surface S, S' from the first direction O1 in one or more second directions 02, ..., On of the plurality of predetermined directions O1, 02, ..., On.

The method 300 then includes a step of acquiring 307, by the electronic processing unit 15, 25, the data representative of the measurements made by said one or more sensors 2, 3, 4, 6 of the endoscope 1 when the external reference surface S, S' of the body 14, 24 of the calibration device 10, 20 is oriented in each of the second directions of the plurality of predetermined directions O1, O2,..., On.

Moreover, it is possible to perform 308, by the electronic processing unit 15, 25, a calibration of the aforesaid one or more sensors 2, 3, 4, 6 of the endoscope 1 based on the acquired data.

In the embodiment in which the aforementioned one or more sensors of the endoscope 1 comprise: an accelerometer 2, a gyroscope 3, one or more magnetic field sensors 4 operatively associated with a permanent magnet 5 housed in the endoscope,
the step of performing a calibration 308 of the one or more sensors of the endoscope 1 comprises the further steps of:
- calculating 401 the orientation of accelerometer 2 with respect to the endoscope 1;
- calculating 402 a bias value of the gyroscope 3;
- calculating 403 a reference magnetic field value generated by said permanent magnet 5 to remove it from the measurements made by the one or more magnetic field sensors 4.

Moreover, when the external reference surface S, S' of the body 14, 24 of the calibration device 10, 20 is oriented in the first direction O1 or in one of the second directions of the plurality of predetermined directions O1, 02,..., On, step 308 of the method 300 includes a step of applying 404 a plurality of different magnetic field values, controlling, by said electronic processing unit 15, 25, the magnetic field source 11, 21.

The step of performing a calibration 308 of the method 300 additionally includes the step of adjusting the sensitivity 405 of each of said one or more magnetic field sensors 4.

Optionally, in the embodiment in which the above-mentioned one or more sensors of the endoscope 1 also comprise the camera 6, the step of performing a calibration 308 of the one or more sensors of the endoscope 1 comprises the further steps of:
- measuring 406 a distortion associated with camera 6;
- calculating 407 the orientation of the camera 6 with respect to the endoscope 1.

Below are some specific examples of the algorithms usable to perform the calibration of the sensors of the endoscope 1.

In a particular embodiment, the aforesaid step of calculating 401 the orientation of the accelerometer 2 with respect to the endoscope 1 of the calibration procedure 308 includes that:
- the endoscope 1 is oriented in a plurality of known predetermined directions O1, 02,..., On, i.e., the calibration device 10, 20 housing such an endoscope 1 is oriented in the first direction O1 and in one of the second directions of the plurality of predetermined directions O1, O2,..., On;
- in each of such predetermined orientation directions O1, 02, ..., On of the plurality, an acceleration measurement provided by the accelerometer 2 is recorded: ACC1, ACC2, ACC3, ..., ACCn.

At this point, an overdetermined linear system is created and solved based on the expression: $\begin{matrix}pseudoinverse \left(\left[\begin{matrix}ACC 1 _ x & ACC 1 _ y & ACC 1 _ z & 1 \\ ACC 2 _ x & ACC 2 _ y & ACC 2 _ z & 1 \\ ACC 3 _ x & ACC 3 _ y & ACC 3 _ z & 1 \\ & ⋮ & & \end{matrix}\right]\right) ∗ \left[\begin{matrix}O 1 \\ O 2 \\ O 3 \\ ⋮\end{matrix}\right] \\ = \left[\begin{matrix}Cal _ xx & Cal _ xy & Cal _ xz \\ Cal _ yx & Cal _ yy & Cal _ yz \\ Cal _ zx & Cal _ zy & Cal _ zz \\ Cal _ qx & Cal _ qy & Cal _ qz\end{matrix}\right]\end{matrix}$ (1) where the resulting matrix of the calibration values is the matrix calculated as output.

It should be noted that, for each orientation direction, it is possible to have several point data.

If multiple samples are taken for each orientation, the calibration value matrix rows on the output can be repeated on the corresponding sample matrix row.

More samples will translate into a higher degree of accuracy. The pseudo-inverse matrix is determined by the fact that the sample matrix is not square. For example, the aforesaid system can be calculated using the Moore-Penrose algorithm or with other pseudo-inversion algorithms such as SVD known to those skilled in the art.

As for the step of calculating 402 a bias value of the gyroscope 3 of the calibration procedure 308, it is assumed that if the endoscope 1 does not move, the angular speeds measured by the gyroscope 3 should be equal to zero. This is generally not true due to such a bias value. In order to remove such a bias value, it is provided that:
- the endoscope 1 is oriented in a plurality of predetermined directions O1, O2,..., On, not known and not fixed, but with a speed equal to zero in each orientation, i.e., that the calibration device 10, 20 housing such an endoscope 1, is oriented in the first direction O1 and in one of the second directions of the plurality of directions O1, O2,..., On;
- in each of such orientation directions O1, 02, ..., On of the plurality, a respective measurement GYRO1, GYRO2, GYRO3, ..., GYROn provided by the gyroscope 3 is recorded;
- three arrays are constructed containing the measurements on each axis (expressed in local coordinates) and the average of the values of each array is calculated as: $\begin{matrix}{GYROBIAS}_{x} = mean \left(GYRO1_x, GYRO2_x, GYRO3_x,…\right) \\ {GYROBIAS}_{y} = mean \left(GYRO1_y, GYRO2_y, GYRO3_y,…\right) \\ {GYROBIAS}_{z} = mean \left(GYRO1_z, GYRO2_z, GYRO3_z,…\right)\end{matrix}$ (2)

The array consists of: $\left[\begin{matrix}{GYROBIAS}_{x} \\ {GYROBIAS}_{y} \\ {GYROBIAS}_{z}\end{matrix}\right]$ (3) represents the set of calibration values of the gyroscope 3.

As for the aforesaid step of calculating 403 a reference magnetic field value generated by the permanent magnet 5 to remove it from the measurements made by the one or more magnetic field sensors 4 of the calibration procedure 308, the following steps are included:
- firstly, it is necessary to ensure that the endoscope 1 is at least 1.5 meters from any magnetic field source in direct current (DC), such as the external permanent magnet 11, 21;
- it is thus possible to orient the endoscope 1 in a plurality of predetermined directions O1, O2,..., On, i.e., that the calibration device 10, 20 housing such an endoscope 1, is oriented in the first direction O1 and in one of the second directions of the plurality of directions O1, O2,..., On, with the aim of mediating the contribution provided by the earth's magnetic field;
- for each of such orientation directions, the values measured by the magnetic field sensors 4 are recorded (there could be a plurality of magnetic field sensors for each axis) : $\begin{matrix}MFS \left(O1\right) = \left[\begin{matrix}{m}_{1} {x}_{1} , {m}_{1} {x}_{2} , … {m}_{1} {x}_{n} , \\ {m}_{1} {y}_{1} , {m}_{1} {y}_{2} , … {m}_{1} {y}_{n} , \\ {m}_{1} {z}_{1} , {m}_{1} {y}_{2} , … {m}_{1} {z}_{n} ,\end{matrix}\right] , \\ MFS \left(O2\right) = \left[\begin{matrix}{m}_{2} {x}_{1} , {m}_{2} {x}_{2} , … {m}_{2} {x}_{n} , \\ {m}_{2} {y}_{1} , {m}_{2} {y}_{2} , … {m}_{2} {y}_{n} , \\ {m}_{2} {z}_{1} , {m}_{2} {y}_{2} , … {m}_{2} {z}_{n} ,\end{matrix}\right] , \\ … \\ MFS \left(Ok\right) = \left[\begin{matrix}{m}_{k} {x}_{1} , {m}_{k} {x}_{2} , … {m}_{k} {x}_{n} , \\ {m}_{k} {y}_{1} , {m}_{k} {y}_{2} , … {m}_{k} {y}_{n} , \\ {m}_{k} {z}_{1} , {m}_{k} {y}_{2} , … {m}_{k} {z}_{n} ,\end{matrix}\right]\end{matrix}$ (4)

For each sensor, it is possible to create an array containing all the recorded data and to average the array as: $\begin{matrix}{OFFSET}_{{x}_{1}} = mean \left({m}_{1}{x}_{1},{m}_{2},{x}_{1},{m}_{3}{x}_{1},…{m}_{k}{x}_{1}\right) \\ {OFFSET}_{{x}_{2}} = mean \left({m}_{1}{x}_{2},{m}_{2},{x}_{2},{m}_{3}{x}_{2},…{m}_{k}{x}_{2}\right) \\ … \\ {OFFSET}_{{x}_{n}} = mean \left({m}_{1}{x}_{n},{m}_{2},{x}_{n},{m}_{3}{x}_{n},…{m}_{k}{x}_{n}\right)\end{matrix}$ (5)

Regarding the aforementioned step of adjusting the sensitivity 405 of each of said one or more magnetic field sensors 4 in the calibration process 308 of the method 300, the following is observed.

As known, the sensitivity of a sensor is a numerical value representing the output unit on Tesla. If the sensors are analog, and the sensor output is a voltage, such a sensitivity is measured for example in Volts/Tesla. In the event of digital sensors, it will be referred to as Least Significant Bit (LSB) on Tesla. In other words, it is possible to define a total sensitivity as: 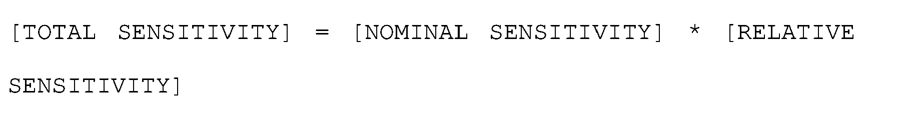 [TOTAL SENSITIVITY] = [NOMINAL SENSITIVITY] * [RELATIVE SENSITIVITY] where the magnitude [RELATIVE SENSITIVITY] is a dimensionless value. If [RELATIVE SENSITIVITY] = 1, the total sensitivity would be equal to the nominal one. In this case:

### [FINAL MEASUREMENT] =

### [RAW MEASUREMENT] * [NOMINAL SENSITIVITY] * [RELATIVE SENSITIVITY]

To calculate the relative sensitivity for each sensor, the solution of an optimization problem is required. In particular:
- the endoscope 1 is positioned in a fixed and unknown position and fixed so as to not move;
- a plurality of known magnetic fields are generated and raw magnetic field values measured by the one or more magnetic field sensors 4 are recorded.

These magnetic fields can be generated by moving an external permanent magnet, e.g., the source 11, 21 or by controlling an electromagnetic device generating different fields. Since the second method can be modeled as an equivalent permanent magnet positioned in a relative position with respect to the endoscope 1, the two methods are equivalent and only the first will be considered below.

The relative sensitivity is calculated by formulating the following optimization problem.

For each measurement N, the problem input data are:
- the position of the external permanent magnet in the global reference system (or equivalent if generated with an electromagnet) - P(n);
- the values measured by the magnetic field sensors 4 - meas (n) .

Therefore, it is possible to write to the following cost function: $J \left({C}_{p}, {C}_{o}, SENS\right) = {\sum }_{i = 1}^{N} B \left(P\left(i\right),{C}_{p},{C}_{o}\right) - \left(meas\left(i\right)∗SENS\right)$ (6) where:
P(i) is the position of the external magnet in the global reference, different for each measurement,
Cp is the unknown and constant position of the endoscope 1 in the global reference system ∈R³
Co is the unknown and constant orientation of the endoscope 1 in the global reference system ∈R³
B(P(i), Cp, Co) is the magnetic field generated by the external magnet in Cp and Co ∈R³
meas(i) - is the array of the sensor measurements given by:$\left[RAW MEASUREMENT\right] * \left[NOMINAL SENSITIVITY\right]$
SENS - is the [RELATIVE SENSITIVITY] array ∈R^{K}, where K is the number of magnetic field sensors 4.

The aim is to find the values of Cp, Co and SENS which minimize J. This can be done with a standard non-linear optimization algorithm such as the matlab fmincon () or python optimization () routines, known to those skilled in the art.

With reference to the aforementioned calibration steps of the camera 6 of the method 300 of the invention, as for the measurement 406 of a distortion associated with the camera 6 and the step 407 of calculating the orientation of the camera 6 with respect to the endoscope 1, the following is provided.

The measurement step 406 of the camera distortion can be performed according to algorithms of known type which include the following operating steps:
- one or more checkerboards are positioned in the field of view of the camera 6, they can be moved or kept stationary;
- the camera images are recorded;
- the calibration algorithm is executed on the recorded images and a matrix representing the calibration of the camera 6 is calculated.

The step of calculating 407 the orientation of the camera 6 with respect to the endoscope 1 includes the following operating steps:
- positioning the endoscope 1 in a device, in particular in the calibration device 10, 20, which forces a known orientation of the endoscope 1 and contains a checkerboard with a known orientation with respect to the orientation of the endoscope 1 and oriented on the plane of the camera 6;
- saving an image acquired by the camera and executing an image processing algorithm to detect the orientation of the aforesaid checkerboard.

Considering the relative orientations expressed as rotation matrices, the rotation of the camera with respect to the endoscope 1, indicated with the variable ${R}_{End}^{Cam}$ can be calculated as: 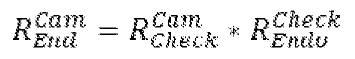 where:
${R}_{Check}^{Cam}$ is a parameter calculated by means of image processing; and
${R}_{Endo}^{Check}$ is a parameter known from the geometry of the device used to calculate the calibration.

The present invention also relates to a calibration device 10, 20 for a calibration system of sensors accommodated in an endoscope 1, such as the devices in figures 2A-2C and 9A-9E.

Such a calibration device comprises a body 14, 24 adapted to house the endoscope 1 and reversible coupling means 16, 16', 26, 26' for reversibly coupling the endoscope 1 to the body 14, 24 of the calibration device 10, 20.

Such reversible coupling means 16, 16', 26, 26' are configured to couple and make the endoscope 1 integral with the body of the calibration device 10, 20 preventing a mutual movement thereof.

Such a body 14, 24 of the calibration device 10, 20 comprises an external reference surface S, S' oriented, with respect to a magnetic field source 11, 21 of the system, in a first direction O1 of a plurality of predetermined directions O1, 02, ..., On.

Moreover, the body 14, 24 of the calibration device 10, 20 is movable to orient in sequence, with respect to the magnetic field source 11, 21, the external reference surface S, S' from the first direction O1 in one or more second directions 02, ..., On of the plurality of predetermined directions O1, 02, ..., On.

The system 100, 200 and the method 300 for calibrating the sensors accommodated in an endoscope 1 usable in a magnetically guided endoscopic system of the present invention have several advantages and achieve the intended objects.

In particular, the suggested system 100, 200 allows calibrating simultaneously and in an integrated manner both the inertial sensors, accelerometer 2 and gyroscope 3, and the magnetic field sensors 4 in the presence of a permanent magnet 5 on board the endoscope 1.

The suggested solution using a calibration device 10, 20 allows performing such a calibration in a simple manner both in the factory, i.e., after manufacturing the endoscope 1, and before using the endoscope, making the calibration device 20 cooperate with a robotic platform 60 through the interface element 70.

Once the endoscope has been calibrated with the system and method of the invention, using the magnetic interaction it is possible to simultaneously manipulate and locate the endoscope during a diagnostic assessment on the patient in a reliable manner.

Those skilled in the art may make changes and adaptations to the embodiments of the method and system described above or can replace elements with others which are functionally equivalent in order to meet contingent needs without departing from the scope of the following claims. Each of the features described above as belonging to one possible embodiment can be implemented irrespective of the other embodiments described.

## Claims

1. A system (100; 200) for calibrating sensors accommodated in an endoscope (1) usable in a magnetically guided endoscopic system, comprising:
- an endoscope (1) including one or more sensors (2, 3, 4, 6) to be calibrated;
- an electronic processing unit (15; 25);
- a magnetic field source (11; 21) controlled by said electronic processing unit (15; 25) to provide a reference magnetic field value to the endoscope (1);
- a reading unit (12; 22) connected to the endoscope (1) and configured to acquire data representative of measurements made by said one or more sensors (2, 3, 4, 6) of the endoscope to be sent to the electronic processing unit (15; 25);
said system (100; 200) further comprising:
- a calibration device (10; 20) having a body (14; 24) adapted to house said endoscope (1);
- reversible coupling means (16, 16'; 26, 26') for reversibly coupling the endoscope (1) to the body (14; 24) of the calibration device (10; 20), said reversible coupling means (16, 16'; 26, 26') being configured to couple and make the endoscope (1) integral with the body of the calibration device (10; 20) preventing a mutual movement thereof;
said body (14; 24) of the calibration device (10; 20) comprising an external reference surface (S; S') oriented, with respect to said magnetic field source (11; 21), in a first direction (O1) of a plurality of predetermined directions (O1, O2,..., On), said body (14; 24) of the calibration device (10; 20) being movable to orient in sequence, with respect to the magnetic field source (11; 21), the external reference surface (S; S') from the first direction (O1) in one or more second directions (O2, ..., On) of the plurality of predetermined directions (O1, O2,..., On),
said electronic processing unit (15; 25) being configured to acquire the data representative of the measurements made by said one or more sensors (2, 3, 4, 6) of the endoscope (1) when the external reference surface (S; S') of the body (14; 24) of the calibration device (10; 20) is oriented both in the first direction (O1) and in each of the second directions of the plurality of predetermined directions (O1, O2, ..., On),
and to perform a calibration of said one or more sensors (2, 3, 4, 6) of the endoscope (1) based on the acquired data.

2. A calibration system (100; 200) according to claim 1, wherein said one or more sensors of the endoscope (1) comprise:
- an accelerometer (2),
- a gyroscope (3),
- one or more magnetic field sensors (4) operatively associated with a permanent magnet (5) housed in the endoscope (1).

3. A calibration system (100; 200) according to claim 1 or 2, wherein said calibration device (10; 20) comprises means (19; 29) for identifying that said external reference surface (S; S') of the body (14; 24) is oriented in said first direction (O1) and in each of said second directions of the plurality of predetermined directions (O1, O2, ..., On).

4. A calibration system (100; 200) according to claim 1 or 2, further comprising a storage unit (13; 23) controlled by said electronic processing unit (15; 25) for storing calibration values associated with said one or more sensors (2, 3, 4, 6).

5. A calibration system (100) according to claim 1 or 2, wherein said reversible coupling means (16, 16') for reversibly coupling the endoscope (1) to the body (14) of the calibration device (10) comprise a mechanism (16) for reversibly locking the endoscope (1) to the body (14) of the calibration device (10) and a mechanism (16') adapted to fix the orientation of the endoscope with respect to the body (14) of the calibration device (10).

6. A calibration system (100) according to claim 1 or 2, wherein said calibration device (10) is housed inside a calibration frame (50) and said magnetic field source (11) controlled by the electronic processing unit (15) comprises a plurality of coils (51) fixed to the frame (50) adapted to provide a reference magnetic field value to the endoscope (1).

7. A calibration system (100) according to claim 1 or 2, wherein said calibration device (10) is connected to a rotation actuation mechanism (55) controlled by the electronic processing unit (15) to allow the automatic rotation of the endoscope (1) about any of said axes so as to take at least three mutually orthogonal positions.

8. A calibration system (100) according to claim 4, wherein said endoscope further comprises a non-volatile type memory (18) and said unit (13) for storing calibration values associated with said one or more sensors (2, 3, 4, 6) comprises a unit (17) for writing said calibration values in said non-volatile memory (18).

9. A calibration system (100) according to claim 4, wherein said unit (13) for storing calibration values associated with said one or more sensors (2, 3, 4, 6) further comprises a unit (40) for generating unique graphic identification codes (41), each including said calibration values in a machine-readable format, said identification codes (41) being printable on an external surface (42) of a packaging (43) of the endoscope (1).

10. A calibration system (100) according to claim 9, wherein said unique graphic identification codes (41) are one-dimensional codes, of the barcode-type, or two-dimensional codes, of the Data Matrix or QR-code type.

11. A calibration system (100) according to claim 3, wherein said calibration device (10) has the shape of a prism having an internal cavity (10') for housing the endoscope (1) delimited by a frame (10a) defining a plurality of mutually orthogonal flat surfaces (10b) of the calibration device (10),
said means (19) for identifying that said external reference surface (S) of the body (14) is oriented in said first direction (O1) and in each of said second directions of the plurality of predetermined directions (O1, O2, ..., On) comprise marks imprinted on at least one of said flat surfaces (10b) of the frame.

12. A calibration system (200) according to claim 3, wherein said calibration device (20) has the prism-shaped body (24) adapted to delimit an internal cavity (10") of the body for housing the endoscope (1), said body (24) defining six mutually orthogonal flat surfaces (20b) of the calibration device (20),
said means (29) for identifying that said external reference surface (S') of the body (24) is oriented in said first direction (O1) and in each of said second directions of the plurality of predetermined directions (O1, O2, ..., On) comprise one or more holes (29) obtained on one or more of said flat surfaces (20b) of the body (24) of the calibration device (20), said one or more holes (29) being configured to engage respective one or more protruding pins (30) associated with a robotic platform (60) of the calibration system (200).

13. A calibration system (200) according to claim 12, further comprising an interface element (70) housed in the robotic platform (60), said interface element (70) comprising said one or more protruding pins (30) adapted to be engaged with said one or more holes (29) of the calibration device (20) to allow coupling the calibration device (20) to the interface element (70).

14. A calibration system (200) according to claim 13, wherein said interface element (70) has a respective prism-shaped body (71) comprising a base wall (72) connected to side walls (73, 74) orthogonal to said base part (72) adapted to delimit a first cavity (75) for inserting the calibration device (20) into the interface element (70), said one or more protruding pins (30) of the interface element (70) being arranged on said base wall (72) to project towards the first cavity (75).

15. A calibration system (100; 200) according to any one of the preceding claims, wherein said one or more sensors of the endoscope (1) further comprise a camera (6).

16. A calibration system (100; 200) according to claim 15, wherein said calibration device (10; 20) comprises, inside the housing body (14; 24) of the endoscope (1), an element (80; 81) for displaying one or more calibration images to be provided to said camera (6) of the endoscope (1).

17. A method (300) for calibrating sensors accommodated in an endoscope (1) usable in a magnetically guided endoscopic system, comprising the steps of:
- providing (301) a system (100; 200) comprising:
- an endoscope (1) including one or more sensors (2, 3, 4, 6) to be calibrated;
- an electronic processing unit (15; 25);
- a magnetic field source (11; 21) controlled by said electronic processing unit (15; 25) to provide a reference magnetic field value to the endoscope (1);
- a reading unit (12; 22) connected to the endoscope (1) and configured to acquire data representative of measurements made by said one or more sensors (2, 3, 4, 6) of the endoscope to be sent to the electronic processing unit (15; 25);
said method (300) further comprising the steps of:
- providing (302) a calibration device (10; 20) having a body (14; 24) adapted to house said endoscope (1);
- coupling and making integral (303) the endoscope (1) with the body (14; 24) of the calibration device (10; 20) preventing a mutual movement thereof by reversible coupling means (16, 16'; 26, 26') for reversibly coupling the endoscope (1) to the body of the calibration device (10; 20);
- orienting (304), with respect to said magnetic field source (11; 21), an external reference surface (S; S') of said body (14; 24) of the calibration device (10; 20) in a first direction (O1) of a plurality of predetermined directions (O1, O2, ..., On);
- acquiring (305), by said electronic processing unit (15; 25), the data representative of the measurements made by said one or more sensors (2, 3, 4, 6) of the endoscope (1) when the external reference surface (S; S') of the body (14; 24) of the calibration device (10; 20) is oriented in the first direction (O1);
- moving (306) the body (14; 24) of the calibration device (10; 20) to orient in sequence, with respect to the magnetic field source (11; 21), the external reference surface (S; S') from the first direction (O1) in one or more second directions (O2, ..., On) of the plurality of predetermined directions (O1, O2, ..., On);
- acquiring (307), by said electronic processing unit (15; 25), the data representative of the measurements made by said one or more sensors (2, 3, 4, 6) of the endoscope (1) when the external reference surface (S; S') of the body (14; 24) of the calibration device (10; 20) is oriented in each of the second directions of the plurality of predetermined directions (O1, O2, ..., On);
- performing (308), by said electronic processing unit (15; 25), a calibration of said one or more sensors (2, 3, 4, 6) of the endoscope (1) based on the acquired data.

18. A method (300) for calibrating sensors accommodated in an endoscope (1) according to claim 17, wherein said one or more sensors of the endoscope (1) comprise:
- an accelerometer (2),
- a gyroscope (3),
- one or more magnetic field sensors (4) operatively associated with a permanent magnet (5) housed in the endoscope (1),
and wherein said step of performing a calibration (308) of said one or more sensors of the endoscope (1) comprises the further steps of:
- calculating (401) the orientation of the accelerometer (2) with respect to the endoscope (1);
- calculating (402) a bias value of the gyroscope (3) ;
- calculating (403) a reference magnetic field value generated by said permanent magnet (5) to remove it from the measurements made by the one or more magnetic field sensors (4);
- when the external reference surface (S; S') of the body (14; 24) of the calibration device (10; 20) is oriented in the first direction (O1) or in one of the second directions of the plurality of predetermined directions (O1, O2, ..., On), applying (404) a plurality of different magnetic field values, controlling, by said electronic processing unit (15; 25), the magnetic field source (11; 21);
- adjusting the sensitivity (405) of each of said one or more magnetic field sensors (4).

19. A method (300) for calibrating sensors accommodated in an endoscope (1) according to claim 18, wherein said one or more sensors of the endoscope (1) further comprise a camera (6), and wherein said step of performing a calibration (308) of said one or more sensors of the endoscope (1) comprises the further steps of:
- measuring (406) a distortion associated with the camera (6);
- calculating (407) the orientation of the camera (6) with respect to the endoscope (1).

## Patentansprüche

1. System (100; 200) zur Kalibrierung von Sensoren, die in einem Endoskop (1) untergebracht sind und in einem magnetisch geführten endoskopischen System verwendet werden können, umfassend:
- ein Endoskop (1) mit einem oder mehreren zu kalibrierenden Sensoren (2, 3, 4, 6);
- eine elektronische Verarbeitungseinheit (15; 25);
- eine Magnetfeldquelle (11; 21), die von der genannten elektronischen Verarbeitungseinheit (15; 25) gesteuert wird, um dem Endoskop (1) einen Referenzwert für das Magnetfeld zur Verfügung zu stellen;
- eine Leseeinheit (12; 22), die an das Endoskop (1) angeschlossen und dazu ausgelegt ist, für von den genannten einem oder mehreren Sensoren (2, 3, 4, 6) des Endoskops durchgeführte Messungen repräsentative und an die elektronische Verarbeitungseinheit (15; 25) zu sendende Daten zu erfassen;
wobei das genannte System (100; 200) außerdem Folgendes umfasst:
- eine Kalibrierungsvorrichtung (10; 20) mit einem Gehäuse (14; 24), das geeignet ist, das genannte Endoskop (1) unterzubringen;
- reversible Kopplungsmittel (16, 16'; 26, 26') zum reversiblen Koppeln des Endoskops (1) mit dem Gehäuse (14; 24) der Kalibrierungsvorrichtung (10; 20); wobei die genannten reversiblen Kopplungsmittel (16, 16'; 26, 26') dazu ausgelegt sind, das Endoskop (1) mit dem Gehäuse der Kalibrierungsvorrichtung (10; 20) zu koppeln und so eine Einheit zu bilden und eine gegenseitige Bewegung derselben zu verhindern;
wobei das genannte Gehäuse (14; 24) der Kalibrierungsvorrichtung (10; 20) eine externe Referenzfläche (S; S') umfasst, die, im Verhältnis zu der genannten Magnetfeldquelle (11; 21), in einer ersten Richtung (O1) einer Vielzahl von vorgegebenen Richtungen (O1, O2, ..., On) ausgerichtet ist, wobei das genannte Gehäuse (14; 24) der Kalibrierungsvorrichtung (10; 20) beweglich ist, um der Reihe nach, im Verhältnis zu der Magnetfeldquelle (11; 21), die externe Referenzfläche (S; S') von der ersten Richtung (O1) in einer oder mehreren zweiten Richtungen (O2, ..., On) der Vielzahl vorgegebener Richtungen (O1, O2, ..., On) auszurichten,
wobei die genannte elektronische Verarbeitungseinheit (15; 25) dazu ausgelegt ist, die für die von dem genannten einen oder den genannten mehreren Sensoren (2, 3, 4, 6) des Endoskops (1) durchgeführten Messungen repräsentativen Daten zu erfassen, wenn die externe Referenzfläche (S; S') des Gehäuses (14; 24) der Kalibrierungsvorrichtung (10; 20) sowohl in der ersten Richtung (O1) als auch in jeder der zweiten Richtungen der Vielzahl vorgegebener Richtungen (O1, O2, ..., On) ausgerichtet ist,
und eine Kalibrierung des genannten einen oder der genannten mehreren Sensoren (2, 3, 4, 6) des Endoskops (1) auf Grundlage der erfassten Daten durchzuführen.

2. Kalibriersystem (100; 200) nach Anspruch 1, wobei der genannte eine oder die genannten mehreren Sensoren des Endoskops (1) Folgendes umfassen:
- einen Beschleunigungsmesser (2),
- ein Gyroskop (3),
- einen oder mehrere Magnetfeldsensoren (4), die operativ mit einem in dem Endoskop (1) untergebrachten Permanentmagneten (5) verbunden sind.

3. Kalibriersystem (100; 200) nach Anspruch 1 oder 2, wobei die genannte Kalibrierungsvorrichtung (10; 20) Mittel (19; 29) umfasst, um festzustellen, ob die genannte externe Referenzfläche (S; S') des Gehäuses (14; 24) in der genannten ersten Richtung (O1) und in jeder der genannten zweiten Richtungen der Vielzahl vorgegebener Richtungen (O1, O2, ..., On) ausgerichtet ist.

4. Kalibriersystem (100; 200) nach Anspruch 1 oder 2, das außerdem eine Speichereinheit (13; 23) umfasst, die von der genannten elektronischen Verarbeitungseinheit (15; 25) gesteuert wird, um mit dem genannten einen oder den genannten mehreren Sensoren (2, 3, 4, 6) verknüpfte Kalibrierwerte zu speichern.

5. Kalibriersystem (100) nach Anspruch 1 oder 2, wobei die genannten reversiblen Kopplungsmittel (16, 16') zum reversiblen Koppeln des Endoskops (1) mit dem Gehäuse (14) der Kalibrierungsvorrichtung (10) einen Mechanismus (16) zum reversiblen Verriegeln des Endoskops (1) am Gehäuse (14) der Kalibrierungsvorrichtung (10) und einen Mechanismus (16') umfassen, der geeignet ist, die Ausrichtung des Endoskops im Verhältnis zum Gehäuse (14) der Kalibrierungsvorrichtung (10) zu fixieren.

6. Kalibriersystem (100) nach Anspruch 1 oder 2, wobei die genannte Kalibrierungsvorrichtung (10) in einem Kalibrierrahmen (50) untergebracht ist und die von der elektronischen Verarbeitungseinheit (15) gesteuerte genannte Magnetfeldquelle (11) eine Vielzahl von Spulen (51) umfasst, die am Rahmen (50) befestigt und geeignet sind, dem Endoskop (1) einen Referenzwert für das Magnetfeld zur Verfügung zu stellen.

7. Kalibriersystem (100) nach Anspruch 1 oder 2, wobei die genannte Kalibrierungsvorrichtung (10) an einen von der elektronischen Verarbeitungseinheit (15) gesteuerten Drehantriebsmechanismus (55) angeschlossen ist, um die automatische Drehung des Endoskops (1) um eine beliebige der genannten Achsen zu gestatten, so dass es mindestens drei zueinander orthogonale Positionen einnehmen kann.

8. Kalibriersystem (100) nach Anspruch 4, wobei das genannte Endoskop außerdem einen Speicher vom nichtflüchtigen Typ (18) umfasst und die genannte Einheit (13) zum Speichern von mit dem genannten einen oder den genannten mehreren Sensoren (2, 3, 4, 6) verknüpften Kalibrierwerten eine Einheit (17) zum Schreiben der genannten Kalibrierwerte in den genannten nichtflüchtigen Speicher (18) umfasst.

9. Kalibriersystem (100) nach Anspruch 4, wobei die genannte Einheit (13) zum Speichern von mit dem genannten einen oder den genannten mehreren Sensoren (2, 3, 4, 6) verknüpften Kalibrierwerten außerdem eine Einheit (40) zum Generieren eindeutiger grafischer Identifikationscodes (41) umfasst, die jeweils die genannten Kalibrierwerte in einem maschinenlesbaren Format enthalten, wobei die genannten Identifikationscodes (41) auf eine Außenfläche (42) einer Verpackung (43) des Endoskops (1) gedruckt werden können.

10. Kalibriersystem (100) nach Anspruch 9, wobei es sich bei den genannten eindeutigen grafischen Identifikationscodes (41) um eindimensionale Codes vom Barcode-Typ oder um zweidimensionale Codes vom Data-Matrix- oder QR-Code-Typ handelt.

11. Kalibriersystem (100) nach Anspruch 3, wobei die genannte Kalibrierungsvorrichtung (10) die Form eines Prismas mit einem inneren Hohlraum (10') zum Unterbringen des Endoskops (1) aufweist, der von einem Rahmen (10a) begrenzt wird, der eine Vielzahl zueinander orthogonaler ebener Flächen (10b) der Kalibrierungsvorrichtung (10) definiert,
wobei die genannten Mittel (19) zur Feststellung, dass die genannte externe Referenzfläche (S) des Gehäuses (14) in der genannten ersten Richtung (O1) und in jeder der genannten zweiten Richtungen der Vielzahl vorgegebener Richtungen (O1, O2, ..., On) ausgerichtet ist, Markierungen umfassen, die auf mindestens einer der genannten ebenen Flächen (10b) des Rahmens eingeprägt sind.

12. Kalibriersystem (200) nach Anspruch 3, wobei die genannte Kalibrierungsvorrichtung (20) ein prismenförmiges Gehäuse (24) aufweist, das geeignet ist, einen inneren Hohlraum (10") zum Unterbringen des Endoskops (1) zu begrenzen, wobei das genannte Gehäuse (24) sechs zueinander orthogonale ebene Flächen (20b) der Kalibrierungsvorrichtung (20) definiert,
wobei die genannten Mittel (29) zur Feststellung, dass die genannte externe Referenzfläche (S') des Gehäuses (24) in der genannten ersten Richtung (O1) und in jeder der genannten zweiten Richtungen der Vielzahl vorgegebener Richtungen (O1, O2, ..., On) ausgerichtet ist, ein oder mehrere Öffnungen (29) umfassen, die auf einer oder mehreren der genannten ebenen Flächen (20b) des Gehäuses (24) der Kalibrierungsvorrichtung (20) eingerichtet sind, wobei die genannte eine oder die genannten mehreren Öffnungen (29) dazu ausgelegt sind, mit jeweils einem oder mehreren vorstehenden Stiften (30), die mit einer Roboterplattform (60) des Kalibriersystems (200) verbunden sind, in Eingriff zu gelangen.

13. Kalibriersystem (200) nach Anspruch 12, das außerdem ein in der Roboterplattform (60) untergebrachtes Schnittstellenelement (70) umfasst, wobei das genannte Schnittstellenelement (70) den genannten einen oder die genannten mehreren vorstehenden Stifte (30) umfasst, die geeignet sind, mit der genannten einen oder den genannten mehreren Öffnungen (29) der Kalibrierungsvorrichtung (20) in Eingriff zu gelangen, um das Koppeln der Kalibrierungsvorrichtung (20) mit dem Schnittstellenelement (70) zu gestatten.

14. Kalibriersystem (200) nach Anspruch 13, wobei das genannte Schnittstellenelement (70) ein entsprechendes prismenförmiges Gehäuse (71) aufweist, das eine Bodenwand (72) umfasst, die mit Seitenwänden (73, 74) verbunden ist, die orthogonal zu dem genannten Bodenteil (72) verlaufen und geeignet sind, einen ersten Hohlraum (75) zum Einsetzen der Kalibrierungsvorrichtung (20) in das Schnittstellenelement (70) zu begrenzen, wobei der genannte eine oder die genannten mehreren vorstehenden Stifte (30) des Schnittstellenelements (70) an der genannten Bodenwand (72) angeordnet sind, um in Richtung des ersten Hohlraums (75) vorzuragen.

15. Kalibriersystem (100; 200) nach einem beliebigen der vorangegangenen Ansprüche, wobei der genannte eine oder die genannten mehreren Sensoren des Endoskops (1) außerdem eine Kamera (6) umfassen.

16. Kalibriersystem (100; 200) nach Anspruch 15, wobei die genannte Kalibrierungsvorrichtung (10; 20) innerhalb des Gehäusekörpers (14; 24) des Endoskops (1) ein Element (80; 81) zur Anzeige eines oder mehrerer Kalibrierbilder umfasst, die der genannten Kamera (6) des Endoskops (1) zur Verfügung gestellt werden sollen.

17. Verfahren (300) zur Kalibrierung von Sensoren, die in einem Endoskop (1) untergebracht sind, das in einem magnetisch geführten endoskopischen System eingesetzt werden kann, umfassend die folgenden Schritte:
- Bereitstellung (301) eines Systems (100; 200) umfassend:
- ein Endoskop (1) mit einem oder mehreren zu kalibrierenden Sensoren (2, 3, 4, 6);
- eine elektronische Verarbeitungseinheit (15; 25);
- eine Magnetfeldquelle (11; 21), die von der genannten elektronischen Verarbeitungseinheit (15; 25) gesteuert wird, um dem Endoskop (1) einen Referenzwert für das Magnetfeld zur Verfügung zu stellen;
- eine Leseeinheit (12; 22), die an das Endoskop (1) angeschlossen und dazu ausgelegt ist, für von den genannten einem oder mehreren Sensoren (2, 3, 4, 6) des Endoskops durchgeführte Messungen repräsentative und an die elektronische Verarbeitungseinheit (15; 25) zu sendende Daten zu erfassen;
wobei das genannte Verfahren (300) außerdem die folgenden Schritte umfasst:
- Bereitstellung (302) einer Kalibrierungsvorrichtung (10; 20) mit einem Gehäuse (14; 24), das geeignet ist, das genannte Endoskop (1) unterzubringen;
- Koppeln und Bilden einer Einheit (303) des Endoskops (1) mit dem Gehäuse (14; 24) der Kalibrierungsvorrichtung (10; 20), so dass eine gegenseitige Bewegung derselben durch reversible Kopplungsmittel (16, 16'; 26, 26') zum reversiblen Koppeln des Endoskops (1) mit dem Gehäuse der Kalibrierungsvorrichtung (10; 20) verhindert wird;
- Ausrichten (304) einer externen Referenzfläche (S; S') des genannten Gehäuses (14; 24) der Kalibrierungsvorrichtung (10; 20), im Verhältnis zu der genannten Magnetfeldquelle (11; 21), in einer ersten Richtung (O1) einer Vielzahl von vorgegebenen Richtungen (O1, O2, ..., On);
- Erfassen (305), durch die genannte elektronische Verarbeitungseinheit (15; 25), der für die von dem genannten einen oder den genannten mehreren Sensoren (2, 3, 4, 6) des Endoskops (1) durchgeführten Messungen repräsentativen Daten, wenn die externe Referenzfläche (S; S') des Gehäuses (14; 24) der Kalibrierungsvorrichtung (10; 20) in der ersten Richtung (O1) ausgerichtet ist;
- Bewegen (306) des Gehäuses (14; 24) der Kalibrierungsvorrichtung (10; 20), um die externe Referenzfläche (S; S') im Verhältnis zu der Magnetfeldquelle (11; 21) der Reihe nach von der ersten Richtung (O1) in eine oder mehrere zweite Richtungen (O2, ..., On) der Vielzahl vorgegebener Richtungen (O1, O2, ..., On) auszurichten;
- Erfassen (307), durch die genannte elektronische Verarbeitungseinheit (15; 25), der für die von dem genannten einen oder den genannten mehreren Sensoren (2, 3, 4, 6) des Endoskops (1) durchgeführten Messungen repräsentativen Daten, wenn die externe Referenzfläche (S; S') des Gehäuses (14; 24) der Kalibrierungsvorrichtung (10; 20) in jeder der zweiten Richtungen der Vielzahl vorgegebener Richtungen (O1, O2, ..., On) ausgerichtet ist;
- Durchführen (308), durch die genannte elektronische Verarbeitungseinheit (15; 25), einer Kalibrierung des genannten einen oder der genannten mehreren Sensoren (2, 3, 4, 6) des Endoskops (1) auf Grundlage der erfassten Daten.

18. Verfahren (300) zur Kalibrierung von Sensoren, die in einem Endoskop (1) untergebracht sind, gemäß Anspruch 17, wobei der genannte eine oder die genannten mehreren Sensoren des Endoskops (1) Folgendes umfassen:
- einen Beschleunigungsmesser (2),
- ein Gyroskop (3),
- ein oder mehrere Magnetfeldsensoren (4), die operativ mit einem in dem Endoskop (1) untergebrachten Permanentmagneten (5) verbunden sind,
und wobei der genannte Schritt der Durchführung einer Kalibrierung (308) des genannten einen oder der genannten mehreren Sensoren des Endoskops (1) die folgenden weiteren Schritte umfasst:
- Berechnen (401) der Ausrichtung des Beschleunigungsmessers (2) - im Verhältnis zum Endoskop (1);
- Berechnen (402) eines Bias-Wertes des Gyroskops (3);
- Berechnen (403) eines Referenzwertes für das Magnetfeld, der von dem genannten Permanentmagneten (5) generiert wird, um diesen aus den von dem einen oder den mehreren Magnetfeldsensoren (4) durchgeführten Messungen zu entfernen;
- wenn die externe Referenzfläche (S; S') des Gehäuses (14; 24) der Kalibrierungsvorrichtung (10; 20) in der ersten Richtung (O1) oder in einer der zweiten Richtungen der Vielzahl vorgegebener Richtungen (O1, O2, ..., On) ausgerichtet ist, Anwendung (404) einer Vielzahl von unterschiedlichen Magnetfeldwerten, wobei die Magnetfeldquelle (11; 21) durch die genannte elektronische Verarbeitungseinheit (15; 25) gesteuert wird;
- Einstellen der Empfindlichkeit (405) jeweils des genannten einen oder der genannten mehreren Magnetfeldsensoren (4).

19. Verfahren (300) zur Kalibrierung von Sensoren, die in einem Endoskop (1) untergebracht sind, nach Anspruch 18, wobei der genannte eine oder die genannten mehreren Sensoren des Endoskops (1) außerdem eine Kamera (6) umfassen und wobei der genannte Schritt der Durchführung einer Kalibrierung (308) des genannten einen oder der genannten mehreren Sensoren des Endoskops (1) die weiteren folgenden Schritte umfasst:
- Messen (406) einer mit der Kamera (6) verbundenen Verzerrung;
- Berechnen (407) der Ausrichtung der Kamera (6) im Verhältnis zum Endoskop (1).

## Revendications

1. Système (100 ; 200) pour étalonner des capteurs logés dans un endoscope (1) utilisable dans un système endoscopique à guidage magnétique, comprenant :
- un endoscope (1) comportant un ou plusieurs capteurs (2, 3, 4, 6) à étalonner;
- une unité de traitement électronique (15 ; 25);
- une source de champ magnétique (11 ; 21) commandée par ladite unité de traitement électronique (15 ; 25) pour fournir une valeur de champ magnétique de référence à l'endoscope (1);
- une unité de lecture (12 ; 22) connectée à l'endoscope (1) et configurée pour acquérir des données représentatives des mesures effectuées par lesdits un ou plusieurs capteurs (2, 3, 4, 6) de l'endoscope à envoyer à l'unité de traitement électronique (15 ; 25);
ledit système (100 ; 200) comprenant en outre:
- un dispositif d'étalonnage (10 ; 20) ayant un corps (14 ; 24) adapté pour loger ledit endoscope (1);
- des moyens de couplage réversible (16, 16'; 26, 26') pour coupler de manière réversible l'endoscope (1) au corps (14 ; 24) du dispositif d'étalonnage (10 ; 20), lesdits moyens de couplage réversible (16, 16'; 26, 26') étant configurés pour coupler et rendre l'endoscope (1) solidaire du corps du dispositif d'étalonnage (10 ; 20) empêchant un mouvement mutuel de celui-ci;
ledit corps (14 ; 24) du dispositif d'étalonnage (10 ; 20) comprenant une surface de référence externe (S ; S') orientée, par rapport à ladite source de champ magnétique (11 ; 21), dans une première direction (O1) d'une pluralité de directions prédéterminées (O1, O2,..., On), ledit corps (14 ; 24) du dispositif d'étalonnage (10 ; 20) étant mobile pour orienter en séquence, par rapport à la source de champ magnétique (11 ; 21), la surface de référence externe (S ; S') à partir de la première direction (O1) dans une ou plusieurs deuxièmes directions (O2, ..., On) de la pluralité de directions prédéterminées (O1, O2,..., On), ladite unité de traitement électronique (15 ; 25) étant configurée pour acquérir les données représentatives des mesures effectuées par ledit ou lesdits capteurs (2, 3, 4, 6) de l'endoscope (1) lorsque la surface de référence externe (S ; S') du corps (14 ; 24) du dispositif d'étalonnage (10 ; 20) est orientée à la fois dans la première direction (O1) et dans chacune des deuxièmes directions de la pluralité de directions prédéterminées (O1, O2, ..., On), et pour effectuer une l'étalonnage dudit ou desdits capteurs (2, 3, 4, 6) de l'endoscope (1) sur la base des données acquises.

2. Système d'étalonnage (100 ; 200) selon la revendication 1, dans lequel ledit ou lesdits capteurs de l'endoscope (1) comprennent :
- un accéléromètre (2),
- un gyroscope (3),
- un ou plusieurs capteurs de champ magnétique (4) associés fonctionnellement à un aimant permanent (5) logé dans l'endoscope (1).

3. Système d'étalonnage (100 ; 200) selon la revendication 1 ou 2, dans lequel ledit dispositif d'étalonnage (10 ; 20) comprend des moyens (19 ; 29) pour identifier que ladite surface de référence externe (S ; S') du corps (14 ; 24) est orientée dans ladite première direction (O1) et dans chacune desdites deuxièmes directions de la pluralité de directions prédéterminées (O1, O2, ..., On).

4. Système d'étalonnage (100 ; 200) selon la revendication 1 ou 2, comprenant en outre une unité de stockage (13 ; 23) commandé par ladite unité de traitement électronique (15 ; 25) pour stocker des valeurs d'étalonnage associées audit ou auxdits capteurs (2, 3, 4, 6).

5. Système d'étalonnage (100) selon la revendication 1 ou 2, dans lequel lesdits moyens de couplage réversible (16, 16') pour coupler de manière réversible l'endoscope (1) au corps (14) du dispositif d'étalonnage (10) comprennent un mécanisme (16) pour verrouiller de manière réversible l'endoscope (1) au corps (14) du dispositif d'étalonnage (10) et un mécanisme (16') adapté pour fixer l'orientation de l'endoscope par rapport au corps (14) du dispositif d'étalonnage (10).

6. Système d'étalonnage (100) selon la revendication 1 ou 2, dans lequel ledit dispositif d'étalonnage (10) est logé à l'intérieur d'un cadre d'étalonnage (50) et ladite source de champ magnétique (11) commandée par l'unité de traitement électronique (15) comprend une pluralité de bobines (51) fixées au cadre (50) adaptées pour fournir une valeur de champ magnétique de référence à l'endoscope (1).

7. Système d'étalonnage (100) selon la revendication 1 ou 2, dans lequel ledit dispositif d'étalonnage (10) est connecté à un mécanisme d'actionnement de rotation (55) commandé par l'unité de traitement électronique (15) pour permettre la rotation automatique de l'endoscope (1) autour de l'un quelconque desdits axes de manière à prendre au moins trois positions mutuellement orthogonales.

8. Système d'étalonnage (100) selon la revendication 4, dans lequel ledit endoscope comprend en outre une mémoire de type non volatile (18) et ladite unité (13) pour stocker des valeurs d'étalonnage associées audit ou auxdits capteurs (2, 3, 4, 6) comprend une unité (17) pour écrire lesdites valeurs d'étalonnage dans ladite mémoire non volatile (18).

9. Système d'étalonnage (100) selon la revendication 4, dans lequel ladite unité (13) pour stocker des valeurs d'étalonnage associées auxdits un ou plusieurs capteurs (2, 3, 4, 6) comprend en outre une unité (40) pour générer des codes d'identification graphiques uniques (41), chacun comprenant lesdites valeurs d'étalonnage dans un format lisible par machine, lesdits codes d'identification (41) pouvant être imprimés sur une surface externe (42) d'un emballage (43) de l'endoscope (1).

10. Système d'étalonnage (100) selon la revendication 9, dans lequel lesdits codes d'identification graphique uniques (41) sont des codes unidimensionnels, du type code à barres, ou des codes bidimensionnels, du type matrice de données ou code QR.

11. Système d'étalonnage (100) selon la revendication 3, dans lequel ledit dispositif d'étalonnage (10) a la forme d'un prisme ayant une cavité interne (10') pour loger l'endoscope (1) délimitée par un cadre (10a) définissant une pluralité de surfaces plates mutuellement orthogonales (10b) du dispositif d'étalonnage (10), lesdits moyens (19) pour identifier que ladite surface de référence externe (S) du corps (14) est orientée dans ladite première direction (O1) et dans chacune desdites deuxièmes directions de la pluralité de directions prédéterminées (O1, O2, ..., On) comprennent des marques imprimées sur au moins l'une desdites surfaces plates (10b) du cadre.

12. Système d'étalonnage (200) selon la revendication 3, dans lequel ledit dispositif d'étalonnage (20) a le corps en forme de prisme (24) adapté pour délimiter une cavité interne (10") du corps pour loger l'endoscope (1), ledit corps (24) définissant six surfaces plates mutuellement orthogonales (20b) du dispositif d'étalonnage (20), lesdits moyens (29) pour identifier que ladite surface de référence externe (S') du corps (24) est orientée dans ladite première direction (O1) et dans chacune desdites deuxièmes directions de la pluralité de directions prédéterminées (O1, O2,..., On) comprennent un ou plusieurs trous (29) obtenus sur une ou plusieurs desdites surfaces plates (20b) du corps (24) du dispositif d'étalonnage (20), lesdits un ou plusieurs trous (29) étant configurés pour engager une ou plusieurs broches saillantes respectives (30) associées à une plate-forme robotique (60) du système d'étalonnage (200).

13. Système d'étalonnage (200) selon la revendication 12, comprenant en outre un élément d'interface (70) logé dans la plate-forme robotique (60), ledit élément d'interface (70) comprenant lesdites une ou plusieurs broches saillantes (30) adaptées pour être engagées avec lesdits un ou plusieurs trous (29) du dispositif d'étalonnage (20) pour permettre le couplage du dispositif d'étalonnage (20) à l'élément d'interface (70).

14. Système d'étalonnage (200) selon la revendication 13, dans lequel ledit élément d'interface (70) a un corps respectif en forme de prisme (71) comprenant une paroi de base (72) connectée à des parois latérales (73, 74) orthogonales à ladite partie de base (72) adaptée pour délimiter une première cavité (75) pour insérer le dispositif d'étalonnage (20) dans l'élément d'interface (70) , lesdites une ou plusieurs broches saillantes (30) de l'élément d'interface (70) étant disposées sur ladite paroi de base (72) pour faire saillie vers la première cavité (75).

15. Système d'étalonnage (100 ; 200) selon l'une quelconque des revendications précédentes, dans lequel lesdits un ou plusieurs capteurs de l'endoscope (1) comprennent en outre une caméra (6).

16. Système d'étalonnage (100 ; 200) selon la revendication 15, dans lequel ledit dispositif d'étalonnage (10 ; 20) comprend, à l'intérieur du corps de boîtier (14 ; 24) de l'endoscope (1), un élément (80 ; 81) pour afficher une ou plusieurs images d'étalonnage à fournir à ladite caméra (6) de l'endoscope (1).

17. Procédé (300) d'étalonnage de capteurs logés dans un endoscope (1) utilisable dans un système endoscopique à guidage magnétique, comprenant les étapes consistant à :
- fournir (301) un système (100 ; 200) comprenant :
- un endoscope (1) comportant un ou plusieurs capteurs (2, 3, 4, 6) à étalonner;
- une unité de traitement électronique (15 ; 25);
- une source de champ magnétique (11 ; 21) commandée par ladite unité de traitement électronique (15 ; 25) pour fournir une valeur de champ magnétique de référence à l'endoscope (1);
- une unité de lecture (12 ; 22) connectée à l'endoscope (1) et configurée pour acquérir des données représentatives des mesures effectuées par lesdits un ou plusieurs capteurs (2, 3, 4, 6) de l'endoscope à envoyer à l'unité de traitement électronique (15 ; 25);
ledit procédé (300) comprenant en outre les étapes consistant à :
- prévoir (302) un dispositif d'étalonnage (10 ; 20) ayant un corps (14 ; 24) adapté pour loger ledit endoscope (1) ;
- coupler et solidariser (303) l'endoscope (1) avec le corps (14 ; 24) du dispositif d'étalonnage (10 ; 20) empêchant un mouvement mutuel de celui-ci par des moyens de couplage réversible (16, 16'; 26, 26') pour coupler de manière réversible l'endoscope (1) au corps du dispositif d'étalonnage (10 ; 20);
- orienter (304), par rapport à ladite source de champ magnétique (11 ; 21), une surface de référence externe (S ; S') dudit corps (14 ; 24) du dispositif d'étalonnage (10 ; 20) dans une première direction (O1) d'une pluralité de directions prédéterminées (O1, O2, ..., On);
- acquérir (305), par ladite unité de traitement électronique (15 ; 25), les données représentatives des mesures effectuées par lesdits un ou plusieurs capteurs (2, 3, 4, 6) de l'endoscope (1) lorsque la surface de référence externe (S ; S') du corps (14 ; 24) du dispositif d'étalonnage (10 ; 20) est orientée dans la première direction (O1);
- déplacer (306) le corps (14 ; 24) du dispositif d'étalonnage (10 ; 20) pour orienter en séquence, par rapport à la source de champ magnétique (11 ; 21), la surface de référence externe (S ; S') à partir de la première direction (O1) dans une ou plusieurs deuxièmes directions (O2, ..., On) de la pluralité de directions prédéterminées (O1, O2, ..., On);
- acquérir (307), par ladite unité de traitement électronique (15 ; 25), les données représentatives des mesures effectuées par ledit ou lesdits capteurs (2, 3, 4, 6) de l'endoscope (1) lorsque la surface de référence externe (S ; S') du corps (14 ; 24) du dispositif d'étalonnage (10 ; 20) est orientée dans chacune des deuxièmes directions de la pluralité de directions prédéterminées (O1, O2, ..., On);
- effectuer (308), par ladite unité de traitement électronique (15 ; 25), un étalonnage dudit ou desdits capteurs (2, 3, 4, 6) de l'endoscope (1) sur la base des données acquises.

18. Procédé (300) pour étalonner des capteurs logés dans un endoscope (1) selon la revendication 17, dans lequel lesdits un ou plusieurs capteurs de l'endoscope (1) comprennent :
- un accéléromètre (2),
- un gyroscope (3),
- un ou plusieurs capteurs de champ magnétique (4) fonctionnellement associés à un aimant permanent (5) logé dans l'endoscope (1), et dans lequel ladite étape consistant à effectuer un étalonnage (308) dudit ou desdits capteurs de l'endoscope (1) comprend les étapes supplémentaires consistant à :
- calculer (401) l'orientation de l'accéléromètre (2) - par rapport à l'endoscope (1);
- calculer (402) une valeur de biais du gyroscope (3);
- calculer (403) une valeur de champ magnétique de référence générée par ledit aimant permanent (5) pour l'éliminer des mesures effectuées par le ou les capteurs de champ magnétique (4);
- lorsque la surface de référence externe (S ; S') du corps (14 ; 24) du dispositif d'étalonnage (10 ; 20) est orientée dans la première direction (O1) ou dans l'une des deuxièmes directions de la pluralité de directions prédéterminées (O1, O2,..., On) , appliquer (404) une pluralité de valeurs de champ magnétique différentes, commander, par ladite unité de traitement électronique (15 ; 25), la source de champ magnétique (11 ; 21);
- ajuster la sensibilité (405) de chacun desdits un ou plusieurs capteurs de champ magnétique (4).

19. Procédé (300) pour étalonner des capteurs logés dans un endoscope (1) selon la revendication 18, dans lequel ledit ou lesdits capteurs de l'endoscope (1) comprennent en outre une caméra (6), et dans lequel ladite étape consistant à effectuer un étalonnage (308) dudit ou desdits capteurs de l'endoscope (1) comprend les étapes supplémentaires consistant à :
- mesurer (406) une distorsion associée à la caméra (6);
- calculer (407) l'orientation de la caméra (6) par rapport à l'endoscope (1).
